(19) Europäisches Patentamt
European Patent Office
Office européen des brevets

(11) EP 0 831 084 A1

# (12) DEMANDE DE BREVET EUROPEEN

(43) Date de publication:
**25.03.1998 Bulletin 1998/13**

(51) Int Cl.6: **C07C 319/04**, C07C 323/52

(21) Numéro de dépôt: **97402126.3**

(22) Date de dépôt: **12.09.1997**

(84) Etats contractants désignés:
**AT BE CH DE DK ES FI FR GB GR IE IT LI LU MC NL PT SE**
Etats d'extension désignés:
**AL LT LV RO SI**

(30) Priorité: **20.09.1996 FR 9611508**
**31.01.1997 FR 9701080**

(71) Demandeur: **ELF AQUITAINE EXPLORATION PRODUCTION FRANCE**
**92400 Courbevoie (FR)**

(72) Inventeur: **Arretz, Emmanuel**
**64000 Pau (FR)**

(74) Mandataire: **Leboulenger, Jean**
**Elf Atochem S.A.,**
**Département Propriété Industrielle,**
**Cedex 42 - La Défense 10**
**92091 Paris la Défense (FR)**

## (54) Procédé de synthèse de l'acide mercapto-3 propionique

(57) Ce procédé de synthèse de l'acide mercapto-3 propionique par réaction d'addition de $H_2S$ à l'acide acrylique est effectué en présence d'un support solide possédant des groupes fonctionnels basiques guanidine, à la condition que ceux-ci soient dépourvus d'hydrogène directement lié à un atome d'azote.

EP 0 831 084 A1

## Description

La présente invention concerne la préparation de l'acide mercapto-3-propionique (MPA), par addition de l'hydrogène sulfuré à l'acide acrylique (AA), selon la réaction (1) :

$$H_2S + CH_2 = CH\text{-}COOH \rightarrow HS\text{-}CH_2\text{-}CH_2\text{-}COOH \qquad (1)$$

Le MPA formé peut réagir sur l'AA présent dans le milieu réactionnel pour donner l'acide thio-3,3'- dipropionique (TDPA) selon la réaction (2) :

$$HS\text{-}CH_2\text{-}CH_2\text{-}COOH + CH_2 = CH\text{-}COOH \rightarrow S(CH_2\text{-}CH_2\text{-}COOH)_2 \qquad (2)$$

Le brevet US 5 008 432 décrit l'addition de $H_2S$ à des composés insaturés tels l'acrylate de méthyle ou l'acide acrylique.

Cette addition est réalisée en présence d'un catalyseur basique choisi parmi l'oxyde de magnésium et les résines échangeuses d'anions basiques. Ces résines sont choisies parmi celles ayant, comme groupe fonctionnel, des amines tertiaires ou des hydroxydes d'ammonium quaternaires.

La réaction a lieu en absence ou en présence de solvants. Ces derniers sont choisis parmi les alcools inférieurs et les hydrocarbures aliphatiques, cycloaliphatiques saturés ou aromatiques.

Si une résine échangeuse d'anion est utilisée, la pression de la réaction est en général de 3037,5 à 6750 kPa.

L'exemple VII décrit l'addition de $H_2S$ à l'acide acrylique, sans solvant, à une pression réactionnelle de 3037,5 kPa, en présence de résine Amberlyst A-21 (Rohm et Haas). Cette résine possède des groupes fonctionnels diméthylamino. Au cours de la réaction il se forme un solide distinct du milieu liquide. Ce solide contient des mercaptans, des sulfures, de l'acide acrylique ainsi que ses dimères et trimères. L'essai N° 4 de cet exemple conduirait pour un rapport molaire $H_2S$/AA de 10,4 à une conversion de 89 % et une sélectivité de 100 % en MPA.

L'essai N°3 conduirait, pour un rapport molaire $H_2S$/AA de 5,4, à une conversion de 90 % pour une sélectivité de 98 % en MPA.

La demande de brevet J07-228568 concerne également la synthèse de MPA par addition de $H_2S$ à l'acide acrylique. Selon le procédé de cette demande, l'addition est réalisée en présence d'une résine échangeuse d'anions et d'un solvant choisi parmi l'eau, les composés amides, esters, éthers ou cétones.

Le solvant amide, ester, éther ou cétone ne doit pas posséder d'hydrogène lié à un atome d'oxygène, de soufre, d'azote et similaire. Parmi les solvants utilisables dans ce procédé on note parmi les solvants amides le diméthylformamide (DMF), le diméthylacétamide, la N-méthylpyrrolidone et la diméthylimidazolidinone, le DMF étant préféré car conduisant à un rendement élevé en MPA.

Parmi les solvants éthers, on note le dioxane, le dioxolane et le diéthylèneglycol diméthyl éther, le dioxane étant préféré car conduisant à un rendement élevé en MPA.

Parmi les cétones, on note l'acétone, la diéthyl cétone, la méthyl éthyl cétone et la méthyl isobutyl cétone.

La résine échangeuse d'anions décrite peut avoir comme groupe fonctionnel une amine tertiaire (résines faiblement basiques) ou un hydroxyde d'ammonium quaternaire (résines fortement basiques). Les résines échangeuses d'anions faiblement basiques sont indiquées comme idéales en pratique car ne formant pas de sels avec les composés du milieu réactionnel.

Le polymère de ces résines rendues insolubles par réticulation, peut être du polystyrène, du polyacrylamide ou une résine époxy.

Dans J07-228568, les exemples de synthèse du MPA mettent en oeuvre les résines suivantes fabriquées par la société Rohm & Haas:

Amberlite IRA 93 (groupes: amine tertiaire)
Amberlite IRA 94 (groupes: amine tertiaire)
Amberlite IRA 900 (groupes: ammonium quaternaire)

L'exemple 2 (résine Amberlite IRA 94) et l'exemple 12 (résine Amberlite IRA 900), réalisés dans des conditions identiques pour ce qui est du ratio molaire ($H_2S$/AA = 3) et de la température réactionnelle égale à 60°C, conduiraient à des rendements en MPA (90,0 % et 89,7 %) et des sélectivités (90,6 % et 90,8 %) quasi identiques.

L'exemple 3, réalisé dans le DMF en présence de résine Amberlite IRA-94 conduirait à une conversion de l'AA de 98,9 %, un rendement de 91,5 % en MPA avec une sélectivité de 92,5 %, pour une rapport molaire $H_2S$/AA de 6,0,

une température réactionnelle de 60°C, une pression de 30 atm (3039 kPa) et une pression maximum de 44 atm (4458 kPa).

Le but de la présente invention est de trouver des conditions de mise en oeuvre de la réaction (1) telles que, tout en gardant un taux de conversion très élevé, la sélectivité en MPA soit notablement meilleure que celle de l'art antérieur, notamment celle que l'on pourrait obtenir à partir de l'enseignement technique du document J07-228568.

Ce but est atteint dans la réaction (1) ci-dessus, en remplaçant les résines de l'art antérieur par un support solide ayant des groupes fonctionnels guanidine, à la condition que ceux-ci soient dépourvus d'hydrogène directement lié à un atome d'azote.

La présente invention a donc pour objet un procédé de préparation de l'acide mercapto-3 propionique par réaction d'addition de $H_2S$ à l'acide acrylique en présence d'un support solide possédant des groupes fonctionnels basiques, caractérisé en ce que les groupes fonctionnels sont des groupes guanidine, à la condition que ceux-ci soient dépourvus d'hydrogène directement lié à un atome d'azote.

Le support solide peut être tout support insoluble dans le milieu réactionnel. Comme exemples de tels supports on peut mentionner la silice et l'alumine, mais on préfère utiliser un support polymérique quelconque.

Lorsque la réaction (1) est mise en oeuvre en présence d'un solvant, le support polymérique doit en outre être sensiblement insoluble dans le solvant. D'une manière générale, cette insolubilité est obtenue par une réticulation du ou des polymères constituant le support polymérique.

Plus précisément, la présente invention propose un procédé de synthèse de l'acide mercapto-3 propionique par réaction d'addition de $H_2S$ à l'acide acrylique en présence d'un support solide possédant des groupes fonctionnels basiques, caractérisé en ce que ces groupes fonctionnels sont choisis parmi :

1°) un radical guanidine de formule générale (C) :

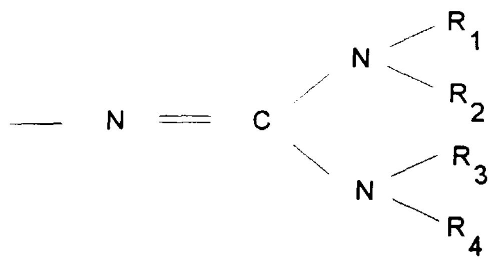

(C)

dans laquelle $R_1$, $R_2$, $R_3$, $R_4$ sont indépendamment l'un de l'autre des groupes hydrocarbonés tels que méthyle, éthyle, propyle, butyle, l'azote imine étant lié au support solide par une liaison chimique ou une succession de liaisons chimiques,

2°) un radical guanidine bicyclique de formule (D) :

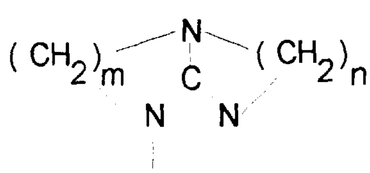

(D)

dans laquelle m et n valent chacun de 2 à 4, avec la condition que n soit inférieur ou égal à m, ce radical (D) étant lié au support solide par une liaison chimique ou une succession de liaisons chimiques portant de l'azote initialement N-H de la guanidine bicyclique correspondante.

Avantageusement, le radical (D) est choisi parmi les radicaux dérivés des guanidines suivantes: 1,5,7-triazabicyclo [4.3.0] non-6-ène (m = 3, n = 2), 1,5,7-triazabicyclo [4.4.0] déc-5-ène (m = 3, n = 3), 1,6,8-triazabicyclo [5.3.0] déc-7-ène (m = 4, n = 2), 1,4,6-triazabicyclo [3.3.0] oct-4-ène (m = 2, n = 2).

Ce procédé permet d'obtenir avec un excellent taux de conversion de l'AA, une meilleure sélectivité en MPA que les procédés de l'art antérieur, avec notamment une diminution concomitante de la teneur en TDPA dans le milieu réactionnel.

Ainsi d'une manière surprenante, tout se passe comme si les groupes fonctionnels guanidines augmentaient sélectivement la cinétique de la réaction (1) par rapport à la cinétique de la réaction (2).

L'augmentation de la sélectivité en MPA du procédé selon la présente invention se base ci-après sur la présentation d'exemples comparatifs comportant un étalonnage quantitatif de chromatogrammes (voir la partie expérimentale).

De préférence, la résine fonctionnalisée à base de polystyrène divinylbenzène (PS-DVB) a la formule générale (I):

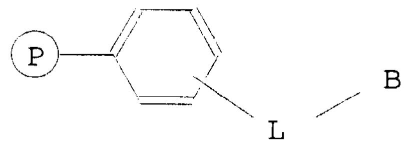

(I)

B étant un groupe choisi parmi les radicaux de formule générale (C) ou (D), L étant un radical organique linéaire ayant une longueur égale ou supérieure à celle du radical méthylène -($CH_2$-) et notamment le radical méthylène,

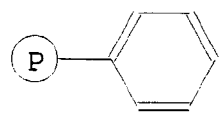

étant le support résine PS-DVB.

De préférence dans la formule générale (I):

- le radical (C) est substitué par L, ce dernier représentant alors un radical -$CH_2$- et $R_1$, $R_2$, $R_3$ et $R_4$ représentant chacun un groupe méthyle,
- le radical (D) est substitué par L sur l'azote qui, dans le composé bicyclique apparenté, porte un hydrogène, L représentant alors un radical -$(CH_2)_p$-, p étant un entier valant 1 à 9.

Avantageusement, la résine polymérique fonctionnalisée a la formule générale (II):

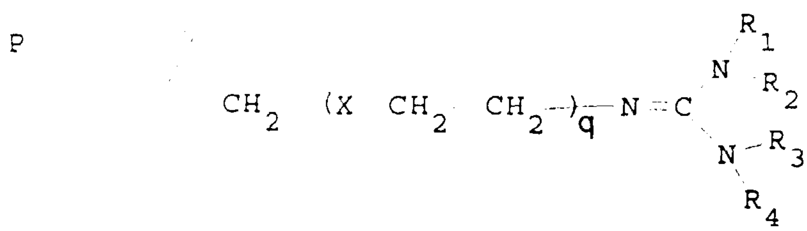

(II)

dans laquelle X représente un atome d'oxygène ou de soufre, q vaut 1 ou 2, $R_1$, $R_2$, $R_3$ et $R_4$ sont indépendamment l'un de l'autre choisis parmi les groupes méthyle, éthyle, propyle, butyle.

Avantageusement, dans la formule générale (II), $R_1$, $R_2$, $R_3$ et $R_4$ représentent chacun un groupe méthyle et q vaut 1.

De préférence, la réaction d'addition (1) a lieu en présence d'un solvant, ce dernier n'ayant pas d'hydrogène mobile.

En général, ledit solvant est un solvant amide, ester, éther ou cétone ou un de leurs mélanges. Avantageusement, ledit solvant est choisi parmi le diméthylformamide (DMF), le diéthylèneglycol diméthyl éther, le dioxane. Le solvant le plus préféré est le DMF.

De préférence, le rapport molaire $H_2S$/AA doit être élevé pour favoriser encore plus la réaction (1) par rapport à la réaction (2). Habituellement ce rapport molaire est compris entre 3 et 10.

Pour augmenter ce rapport molaire dans le milieu liquide en contact avec la résine solide agissant comme catalyseur basique, on préfère soumettre le milieu réactionnel à une pression de $H_2S$ supérieure à la pression atmosphérique. La pression est habituellement supérieure à 15 bars (1500 kPa) et peut atteindre 35 bars (3500 kPa) lorsque la réaction est effectuée à des températures élevées.

Avantageusement, la réaction est effectuée à une température de 20°C à 150°C. De préférence la température du milieu réactionnel va de 30°C à 110°C.

Avantageusement, la quantité en poids de résine utilisée par rapport à la quantité en poids d'acide acrylique mis en oeuvre est de 1 à 100 % et de préférence de 10 à 70 %.

Les catalyseurs à fonction guanidine de l'invention montrent une grande stabilité chimique et thermique vis à vis du milieu réactionnel et ainsi peuvent être utilisées en permanence ou réutilisées sans réactivation.

La réaction peut être conduite dans une réacteur agité ou tubulaire, suivant un procédé discontinu, soit par chargement des réactifs avant leur mise en réaction, soit par addition progressive de l'acide acrylique après l'addition de

l'hydrogène sulfuré, soit encore par addition simultanée des réactifs dans le réacteur, et enfin suivant un procédé continu avec addition contrôlée des réactifs

Les résines de formule générale (I) peuvent être obtenues ou préparées de la manière suivante :

1°) Le groupe B est un radical de formule générale (C).

On connaît du brevet US 5340380 un procédé consistant à substituer le chlore d'une résine chlorométhylée de polystyrène-divinylbenzène par une guanidine substituée ou non et permettant d'obtenir des résines de formule générale (I.C) :

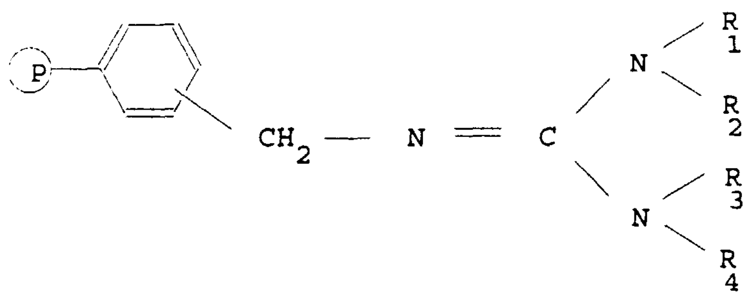

(I.C, L= $CH_2$)

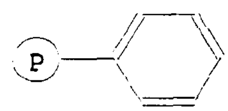

représentant le support solide, résine polystyrène-divinylbenzène de départ, et les symboles $R_1$, $R_2$, $R_3$ et $R_4$ pouvant être chacun un hydrogène, un groupe alkyle ou un groupe aromatique.

Le brevet US 3 346 516 décrit aussi cette réaction d'une résine polystyrène-divinylbenzène chlorométhylée avec la guanidine ou la tétraméthyl guanidine en présence d'un alcool inférieur et un solvant de gonflement du copolymère PS-DVB comme le tétrahydrofuranne, le dioxane ou le diglyme.

Dans le brevet US 5 028 259, la tétraméthylguanidine est mise en contact avec une résine chlorométhylée de polystyrène-divinylbenzène dans un mélange de toluène et de tétrahydrofuranne.

Dans le brevet US 5 340 380 des guanidines sont mises en réaction avec ce même type de résines chlorométhylées en présence de soude dans un solvant constitué d'éthanol ou d'eau.

Cependant cette technique de fonctionnalisation d'une résine PS-DVB chlorométhylée par une guanidine est très limitée dans la pratique pour l'obtention de résines de formules (I.C) dont les radicaux guanidines portent des substituants R1 à $R_4$ différents de quatre méthyles, dans la mesure où seule la 1,1,3,3-tétraméthylguanidine est actuellement commerciale.

De telles résines (I.C) dans lesquelles les groupes $R_1$ à $R_4$ sont tous différents de l'hydrogène peuvent être obtenues en utilisant des urées tétrasubstituées, souvent commercialisées, dans les conditions de préparation suivantes:

a) On commence par préparer une résine PS-DVB fonctionnalisée par des groupes amine primaire et ayant la formule générale (A)

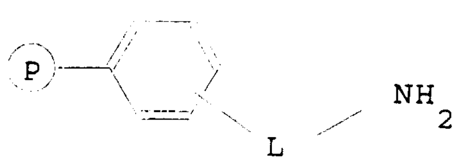

(A)

Celles-ci peuvent être obtenues par différentes techniques:

1/ On peut par exemple partir d'une résine de formule générale (J) :

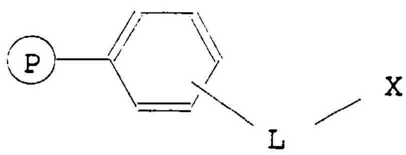

(J)

X étant un groupe partant notamment halogène ou tosylate obtenu à partir d'un groupe hydroxyle -OH, et L représentant notamment un radical $-(CH_2)_p-$, avec p entier valant de 1 à 9.

De préférence, lorsque L représente un seul méthylène, X est un atome de chlore. Dans ce cas, une méthode, décrite par D.H. Rich et S.K. Gurwara, J. Am. Chem. Soc., 1975, 97 - 1575-1579, consiste à faire réagir une résine PS-DVB chlorométhylée avec un excès d'ammoniac. Une autre voie est basée sur l'obtention de résine PS-DVB phtalimidométhylée qui est transformée par hydrazinolyse en résine à fonction amine primaire. Les deux méthodes d'accès à de telles résines phtalimido-méthylées sont décrites dans la publication de A.R. Mitchell, S.B.H. Kent, B.W. Erickson et R.B. Merrifield, Tetrahedron Letters N° 42, 1976, 3795-3798. L'une consiste à partir d'une résine PS-DVB qui par réaction avec le N-(chlorométhyl) phtalimide est directement convertie en résine phtalimidométhylée. L'autre méthode part d'une résine PS-DVB chlorométhylée qui est traitée par le phtalimide de potassium pour donner la résine correspondante phtalimidométhylée.

Quelques résines PS-DVB à fonction amine primaire de formule (A) dans laquelle L représente un méthylène sont commerciales.

Ainsi la Société PUROLITE propose deux résines macroporeuses, l'A-107 et l'A-109, tandis que la Société FLUKA a, dans son catalogue 1995-1996, deux résines gels: la résine 08564 PS réticulée avec 2% de DVB et contenant 1,1 mmole de groupe $-NH_2$ par gramme de résine, et la résine 08566 PS réticulée avec 1 % de DVB et contenant 0,6 mmole de $-NH_2$ par gramme de résine.

La méthode au phtalimide de potassium est également applicable aux résines de formule (J) dans le cas où L est un radical organique linéaire d'une longueur supérieure à celle du radical méthylène, notamment $-(CH_2)_r-$, avec r valant un nombre entier supérieur à 1.

2/ On peut également partir d'une résine PS-DVB de formule (J) dans laquelle L représente un méthylène et X a la signification ci-dessus et de préférence représente un atome de chlore. La Demanderesse a trouvé que cette résine chlorométhylée peut être mise en réaction avec une alkanolamine ou une mercaptoalkylamine, sous forme d'alcoolate ou de thiolate alcalin, dans les conditions de la réaction de Williamson.

Si on met en oeuvre l'éthanolamine, on obtient des résines PS-DVB à fonction amine primaire avec des groupes fonctionnels $-CH_2-O-CH_2-CH_2-NH_2$ fixés aux supports résines PS-DVB.

De manière analogue, en partant du 2-amino éthanethiol hydrochlorure, on obtient les groupes fonctionnels $-CH_2-S-CH_2-CH_2-NH_2$.

Si on utilise le 2-(2-aminoéthoxy)éthanol, on obtient des résines PS-DVB à fonction amine primaire avec des groupes fonctionnels $-CH_2(-O-CH_2-CH_2)_2-NH_2$.

Enfin, en utilisant le 2-[(2-aminoéthyl)thio]-éthanethiol, on obtient des groupes fonctionnels

$-CH_2-(S-CH_2-CH_2)_2-NH_2$.

Cette mercaptoalkylamine de départ peut être préparée selon Iwakura et al., J. Polym. Sci. Part A, 2, 1964, 881-883 ou selon I Voronkov, M.G. et al., Chem. Heterocycl. Compd. (Engl. Transl.)15, 1979, 1183-1185.

Les conditions générales de la réaction de Williamson sont les suivantes :

L'alcanolamine ou la mercaptoalkylamine diluée dans du tétrahydrofuranne (THF) anhydre ou de la N-méthylpyrrolidone anhydre, est mise à réagir avec de l'hydrure de sodium en suspension dans le même solvant anhydre. Après formation de l'alcoolate de sodium ou du thiolate de sodium, la résine chlorométhylée est introduite dans le milieu réactionnel liquide.

b) Après obtention de la résine possédant des groupes amine primaire de formule générale (A), on fait réagir ces groupes amine primaire avec du chlorure de chloroformamidinium (sel de Vilsmeier) de formule générale (H) :

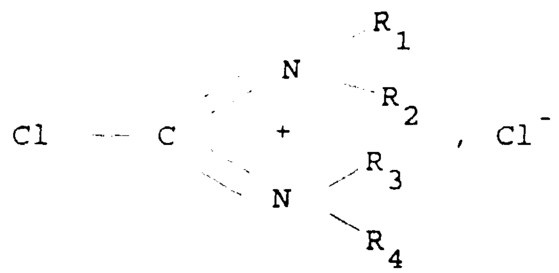

(H)

dans laquelle $R_1$, $R_2$, $R_3$ et $R_4$ sont indépendamment l'un de l'autre choisis parmi les groupes méthyle, éthyle, propyle, butyle,

pour obtenir une résine PS-DVB fonctionnalisée par un groupe guanidine et de formule générale (I.C) :

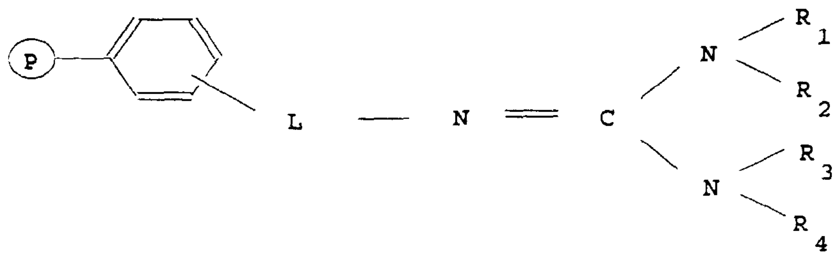

(I.C)

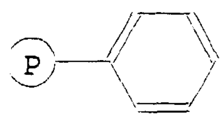

,

L, $R_1$ à $R_4$ ayant les mêmes significations que ci-dessus.

Les chlorures de chloroformamidinium (H) sont généralement obtenus à partir d'urées tétrasubstituées par réaction avec des composés électrophiles tels que phosgène, chlorure de thionyle, chlorure d'oxalyle, oxychlorure de phosphore, suivant des méthodes décrites dans la littérature, notamment:

| | |
|---|---|
| $COCl_2$ | H. Eilingsfeld, M. Seefelder, Angew.Chem., 72, 1960, 836. |
| $SOCl_2$ | H. Ulrich, A.A.R. Sayigh, Angew. Chem. Intern. Ed. Engl., 5, 1966, 704. |
| $(COCl)_2$ | T. Fujisawa et al., Chem. Lett.,1982, 1891. |
| $POCl_3$ | H. Bredereck, K. Bredereck, Chem. Ber., 94, 1961, 2278. |

Généralement, on part de quantités stoechiométriques d'urées tétrasubstituées et de composés chlorés électrophiles et on opère en présence d'un solvant tel que le tétrachlorure de carbone dans le cas du chlorure d'oxalyle, soit sans solvant avec le phosgène ou le chlorure de thionyle, à une température généralement de 0°C à 40°C, et en atmosphère anhydre pour éviter toute hydrolyse.

Avantageusement, on choisit les urées tétrasubstituées parmi la tétraméthylurée, la tétraéthylurée, la tétra n-propylurée et la tétra n-butylurée.

Les chlorures de chloroformamidinium (H) sont mis généralement dans un solvant tel que le toluène ou l'acétonitrile. Leurs réactions avec les résines à fonction amine primaire (A) sont effectuées en présence d'une base, de préférence en présence d'un excès de base.

Si la base est la triéthylamine (TEA), on opère généralement avec un excès molaire de TEA de 10 à 50 % par rapport aux chlorures de chloroformamidinium (H). Ces derniers sont généralement en excès molaire de 10 à 100 % par rapport au nombre de moles de fonction amine primaire, pour transformer la totalité de ces dernières en fonction guanidine.

2°) Dans la formule générale (I) le groupe B est un radical de formule (D)

(a) On commence par préparer une résine de formule générale (J) comme au point 1° a) ci-dessus, L représentant un radical $-(CH_2)_p-$, p entier valant 1 à 9 et X étant un chlore ou un brome.
(b) La résine halogénée ci-dessus est mise en réaction avec une guanidine bicyclique choisie notamment parmi le 1,5,7-triazabicyclo[4.3.0]non-6-ène (m = 3, n = 2), le 1,5,7-triazabicyclo[4.4.0]déc-5-éne (TBD) (m = 3, n = 3), le 1,6,8-triazabicyclo[5.3.0] déc-7-ène (m = 4, n = 2), le 1,4,6-triazabicyclo[3.3.0]oct-4-éne (m = 2, n = 2).

La préparation de ces guanidines bicycliques est décrite dans les brevets GB 826 837 et EP 0 198 680.

La réaction est conduite d'une manière analogue au procédé de M. Tomoi et al, J.M.S. Pure Appl. Chem. A29(3), 1992, 249-261, en particulier page 251 (" Preparation of Polystyrene-Supported TBD).

On obtient ainsi une résine PS-DVB fonctionnalisée par un groupe guanidine bicyclique de formule générale (I.D.) :

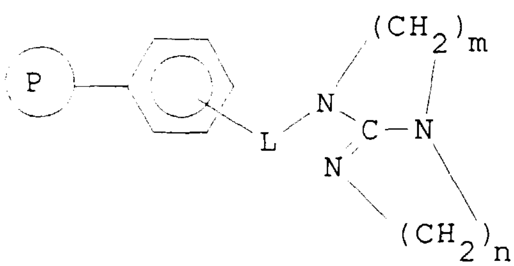

(I.D)

L représentant un radical $-(CH_2)_p-$ avec p entier valant 1 à 9.

Le procédé de Tomoi et al, J. Macromol. Sci. Pure Appl. Chem. A29(3) 1992, 249-261, consiste à faire réagir le sel de lithium du TBD sur une résine chlorométhylée. Un mode opératoire simplifié a été étudié dans le cadre de la présente invention pour préparer des quantités plus importantes de résines à fonction -TBD en faisant réagir directement le 1,5,7-triaza-bicyclo [4.4.0] déc-5-éne en excès sur de la résine PS-DVB chlorométhylée dans du THF anhydre comme solvant.

L'efficacité catalytique des résines utilisées dans la présente invention se trouve améliorée lorsqu'on les utilise sèches.

La présente invention sera mieux comprise grâce à la partie expérimentale ci-après, qui comprend notamment une description de l'appareillage utilisé, ce dernier étant schématiquement représenté sur la figure unique annexée.

## PARTIE EXPERIMENTALE

### I. Préparation de résines polystyrène-divinylbenzène à fonction guanidine.

La résine de base PS-DVB chlorométhylée qui est utilisée est de type macroporeux. Elle a les caractéristiques suivantes :

Surface spécifique : 22,5 $m^2/g$ de résine
Diamètre moyen des pores : 20 Å
Volume des pores : 69 %

chlorométhylée avec un taux de chlore de 19,32 % en poids par rapport au poids total.

Cette résine contient donc 5,44 méq de Cl/g de résine.

*I.1 Obtention de résines PS-DVB de formule (I.C) à fonction 1,1,3,3-tétraméthyl-guanidine (TMG),*

$$(L = -CH_2-, R_1 = R_2 = R_3 = R_4 = CH_3-).$$

La technique utilisée consiste à incorporer directement à une résine PS-DVB chlorométhylée la TMG, selon la méthode décrite dans les brevets US 3 346 516 et 5 028 259.

Mode opératoire:

On pèse 20 g de résine chlorométhylée sèche (5,44 méq de Cl/g de résine). Elle contient 0,109 mole de Cl. On la met en contact sous atmosphère d'azote avec 50 g (0,435 mole) de TMG dilués dans 210 g de tétrahydrofuranne (THF) préalablement séché sur tamis moléculaire. Le milieu réactionnel ainsi obtenu est agité mécaniquement pendant 48 heures à une température de 60°C. Après refroidissement à 20°C, on filtre la résine et on la lave avec 500 ml d'eau,

puis avec 250 ml d'eau à 60°C. On la traite ensuite avec 300 ml d'une solution aqueuse de soude à 10 % et on lave à l'eau jusqu'à neutralité. La résine est lavée au méthanol (300 ml) puis séchée sous vide à 60°C à poids constant.

On a effectué l'analyse élémentaire de la résine ainsi obtenue. Pour cette résine, N = 9,3 % en poids, soit 2,2 mmoles de fonction TMG/g de résine.

Résine indiquée par la suite: PS-DVB-TMG

*I.2 Obtention de résines PS-DVB de formule (I.D.) à fonction 1,5,7-triazabicyclo[4.4.0] déc-5-ène (TBD) avec L = -CH2-.*

Mode opératoire:

On pèse 20 g de résine chlorométhylée sèche (5,44 méq de Cl/g de résine). La charge de résine contient 0,109 mole de Cl. On la met en contact sous atmosphère d'azote avec 30 g (0,216 mole) de TBD dilués dans 285 g de THF préalablement séché sur tamis moléculaire. Le milieu réactionnel ainsi obtenu est agité mécaniquement pendant 48 heures à une température de 60 °C. Après refroidissement à 20 °C, on filtre la résine et on la lave avec 500 ml d'eau, puis avec 250 ml d'eau à 60°C. On la traite ensuite avec 300 ml d'une solution aqueuse de soude à 10 % et on lave à l'eau jusqu'à neutralité. La résine est lavée au méthanol (300 ml) puis séchée sous vide à 60°C à poids constant.

On a effectué l'analyse élémentaire de la résine ainsi obtenue. Pour cette résine, N = 13,26 % en poids, soit 3,15 mmoles de fonction TBD/g de résine.

Résine indiquée par la suite: PS-DVB-TBD

**II Exemples de synthèse d'acide mercapto-3-propionique**

*II.1. Généralités*

Les essais ont été effectués dans un appareillage permettant d'étudier sous pression la réaction de formation de l'acide mercapto-3 propionique à partir de l'acide acrylique et de l'hydrogène sulfuré dans un solvant (le diméthylformamide ou le diglyme) avec différentes résines basiques comme catalyseurs.

L'introduction des réactifs et du solvant avant le démarrage de la réaction permet d'effectuer la réaction selon un procédé batch (l'évolution de la réaction est équivalente aux conditions de fonctionnement en régime piston continu).

La conception de l'appareillage (description de l'appareillage: paragraphe 11.2) permet d'étudier la réaction en régime batch agité (réacteur fermé) au moyen d'un réacteur tubulaire (résine en lit fixe) à travers lequel recircule à grand débit le milieu réactionnel liquide par l'intermédiaire d'une pompe sur le circuit d'une boucle de circulation qui est reliée aux deux extrémités du réacteur.

Cette technique opératoire de type batch agité permet d'étudier la cinétique de la réaction dans des conditions équivalentes à des conditions de fonctionnement en régime piston continu (réacteur ouvert), étant donné que la totalité des réactifs ($H_2S$ et acide acrylique) est introduite, ainsi que le solvant, dans l'appareillage, avant le démarrage de la réaction, le réacteur étant isolé (pas de contact avec la résine) (protocole opératoire : paragraphe II.3).

Le suivi de l'évolution de la réaction au cours du temps est effectué par prélèvement d'échantillons qui sont analysés par chromatographie en phase gazeuse pour déterminer la conversion de l'acide acrylique et les sélectivités correspondantes en acide mercapto-3 propionique et en acide thio-3,3' dipropionique en fonction du temps.

*II.2 Appareillage.*

Comme représenté à la figure unique, l'appareillage en acier inoxydable se compose des éléments suivants :

- un réacteur tubulaire vertical 1, dans lequel est contenu la charge de résine fonctionnalisée 2,
- une boucle de recirculation 3 débouchant à l'extrémité supérieure 4 et à l'extrémité inférieure 5 du réacteur 1, cette boucle comprenant des conduits reliant successivement à partir de l'extrémité inférieure 5, une vanne de fermeture 6, une dérivation 7 équipée d'une vanne 8, un échangeur 9 à double enveloppe, une pompe à engrenage 10 (débit maximum 40 l/h), une prise de température 11, un débit mètre à bille 12, un réservoir cylindrique 13 à double enveloppe, muni d'un hublot 14 en verre épais transparent. Ce réservoir 13 est relié à l'extrémité supérieur 4 du réacteur par un conduit passant par une vanne de fermeture 15. Le réservoir 13 est placé au dessus du réacteur 1.

Cette boucle de circulation 3 comporte elle-même une boucle de dérivation 16 équipée de 2 vannes 17 et 18.

Cette boucle 16 permet d'isoler le réacteur 1 du courant de circulation grâce à la coopération des vannes 6, 15, 17, 18.

Le réservoir 13 est équipé à sa partie supérieure d'un conduit 19 d'introduction de l'acide acrylique et des différents solvants. Ce conduit 19 comporte une vanne 20. Le réservoir 13 est également équipé d'un conduit 21 équipé d'un

vanne de pression 22. Le conduit 21 est relié à une torche.

Le réservoir 13 est équipé à sa partie inférieure d'un conduit 23 équipé d'une vanne 24. et destiné à l'introduction de $H_2S$ sous pression dans le réservoir.

La partie inférieure du réservoir est reliée par un conduit 25 équipé d'une vanne 26 à un récepteur 27. Ce dernier est muni à sa partie inférieure d'un conduit 28 équipé d'une vanne 29. Le conduit 28 permet la récupération d'échantillons en cours de réaction.

*II.3. Protocole opératoire*

Les opérations de chargement de résine et d'introduction de l'acide acrylique et du solvant sont effectuées en atmosphère d'azote.

11.3.1 PREPARATION DES MELANGES REACTIONNELS

Le réacteur 1, contenant la résine 2 (charges de l'ordre de 20 g), est isolé du reste de l'appareillage par la fermeture des vannes 6 et 15. L'acide acrylique et le solvant sont introduits par le conduit 19 dans le réservoir cylindrique 13 qui est en communication directe avec la boucle de recirculation. L'appareillage est mis à une pression de 3 bars d'azote. Le réservoir cylindrique 13, dans lequel va être constitué le mélange réactionnel de départ est refroidi par une circulation d'huile à 12 °C (provenant d'un cryothermostat) qui traverse aussi la double enveloppe externe de l'échangeur 9 de la boucle de recirculation. La pompe de circulation 10 est mise en route et le liquide contenu dans le réservoir cylindrique circule dans la boucle 3 et la boucle 16 en se dirigeant du réservoir 13 vers la vanne 17 avant de passer par la vanne 18.

L'hydrogène sulfuré, provenant d'une alimentation sous 16 bars de pression est injecté par le conduit 23 dans le réservoir 13 au moyen d'un diffuseur et se dissout dans le mélange liquide refroidi (au départ: acide acrylique + solvant). A la fin de l'injection de l'$H_2S$ la pression est de 15 bars et la température du mélange liquide (acide acrylique + $H_2S$ + solvant) est de 20°C. Le volume de chargement peut être contrôlé par le hublot 14.

11.3.2 CONDUITE DES ESSAIS

La consigne du cryothermostat est mise à la valeur correspondant à la température à laquelle la réaction doit être effectuée, et tandis que l'huile monte rapidement à la température fixée, la mélange réactionnel circulant est introduit par l'ouverture des vannes 15 et 6 et la fermeture des vannes 17 et 18 dans le réacteur 1 à travers lequel il recircule à débit élevé (maximum : 40 l/h). La température de réaction programmée pour l'essai est maintenue pendant la durée de la réaction, soit généralement 6 heures. La pression de la phase gazeuse dans l'installation qui est reliée à la vanne de pression 22, s'établit entre 19 bars et 24 bars, suivant les conditions d'essai.

En cours d'essai, à des temps déterminés, des échantillons du milieu réactionnel sont prélevés par l'intermédiaire du récepteur 27 et récupérés à pression atmosphérique, puis analysés par chromatographie en phase gazeuse. A la fin de l'essai l'installation est décomprimée et le produit final de réaction est récupéré.

*II.4.Analyse des produits de réaction*

L'analyse chromatographique en phase gazeuse (CG) a nécessité une mise au point particulière pour résoudre les problèmes posés par l'analyse de l'acide thio-3,3' dipropionique et par la séparation de l'acide acrylique et du diméthylformamide.

L'acide thio-3,3' dipropionique, du fait de ses propriétés physiques et de ses groupes fonctionnels polaires, ne peut être analysé qu'à haute température avec une colonne chromatographique de stabilité thermique élevée (supérieure à 300 °C) et de très faible polarité. Les colonnes de chromatographie qui peuvent convenir sont des colonnes capillaires contenant des phases à base de polysiloxanes ; la phase réticulée diméthylpolysiloxane, non polaire, convient très bien. Ce type de phase n'est pas adapté pour la séparation de l'acide acrylique et du diméthylformamide avec des colonnes couramment utilisées dans les laboratoires c'est à dire des colonnes de 25 mètres ou de 50 mètres. La séparation de ces deux composés a pu être obtenue en connectant en série deux colonnes capillaires Hewlett Packard Ultra-1, respectivement de 50 m et de 25 m, l'appareil de chromatographie étant un Hewlett Packard 5890 FID.

Les résultats des analyses chromatographiques des échantillons de réaction ont été contrôlés par des analyses d'échantillons de référence de compositions pondérales connues, faits à partir d'acide acrylique (AA), d'acide mercapto-3 propionique (MPA), d'acide thio-3,3' dipropionique (TDPA) et de solvant (DMF ou diglyme). Ces analyses de contrôle ont permis de déterminer les facteurs de réponse relatifs aux différents constituants. Dans le cas de l'évaluation de la teneur en acide thio-3,3' dipropionique, l'analyse GC minimise fortement sa teneur réelle dans les échantillons, et une correction précise est indispensable pour déterminer quantitativement l'acide thio-3,3' dipropionique produit dans la réaction.

*II.5 Essais expérimentaux*

II.5.1 GENERALITES

Les essais ont été effectués selon le protocole opératoire que nous avons mis en oeuvre et qui a été décrit dans un paragraphe précédent. Des prélèvements du milieu réactionnel ont été faits à des temps déterminés de chaque essai : au bout de 2 heures, de 4 heures, de 6 heures.

Les échantillons prélevés ont été analysés par chromatographie en phase gazeuse avec la méthode qui a été mise au point. Ces analyses donnent la valeur de la conversion de l'acide acrylique (AA) aux temps déterminés (2 h, 4 h, 6 h) et la sélectivité pondérale en acide mercapto-3 propionique (MPA) et en acide thio-3,3' dipropionique (TDPA).

11.5.2 ESSAIS COMPARATIFS AVEC LA RESINE IRA 94 ET LE DMF

Essai comparatif n° 1

Les conditions opératoires prises pour cet essai correspondent à l'exemple 2 de J07,228,568 :

| | |
|---|---|
| Charge de résine IRA 94 | 24 g |
| DMF | 150 g |
| Acide acrylique | 100 g (1,39 mole) |
| $H_2S$ | 142 g (4,2 moles) |
| Ratio molaire | $\frac{H_2S}{AA} = \frac{3}{1}$ |
| Température | 60 °C |

A la température de réaction de 60 °C, la pression dans l'appareillage est de 20 bars (relatifs).

Essai comparatif n° 2

Les conditions opératoires prises pour cet essai correspondent à l'exemple 3 de J07,228,568 :

| | |
|---|---|
| Charge résine IRA 94 | 24 g |
| DMF | 150 g |
| Acide acrylique | 100 g (1,39 mole) |
| $H_2S$ | 284 g (8,34 moles) |
| Ratio molaire | $\frac{H_2S}{AA} = \frac{6}{1}$ |
| Température | 60°C |

A la température de réaction de 60 °C, la pression dans l'appareillage est de 24 bars.

II.5.3 ESSAIS AVEC LA RESINE PS-DVB-TMG ET LE DMF

Essai n° 3

A l'exception de la résine mise en oeuvre, les conditions opératoires de cet essai sont identiques à celles utilisées pour l'essai n° 1: Charge de résine

| | |
|---|---|
| PS-DVB-TMG | 19 g |
| DMF | 150 g |
| Acide acrylique | 100 g (1,39 mole) |
| $H_2S$ | 142 g (4,2 moles) |
| Ratio molaire | $\frac{H_2S}{AA} = \frac{3}{1}$ |
| Température | 60 °C |

A la température de réaction de 60 °C, la pression dans l'appareillage et de 19 - 20 bars (relatifs).

Essai n° 4

A l'exception de la résine mise en oeuvre, les conditions opératoires de cet essai sont identiques à celles utilisées pour l'essai n° 2:

| | |
|---|---|
| Charge de résine PS-DVB-TMG | 19 g |
| DMF | 150 g |
| Acide acrylique | 100 g (1,39 mole) |
| $H_2S$ | 284 g (8,34 moles) |
| Ratio molaire | $\frac{H_2S}{AA} = \frac{6}{1}$ |
| Température | 60°C |

A la température de réaction de 60 °C, la pression dans l'appareillage est de 23 - 24 bars.

11.5.4 ESSAIS AVEC LA RESINE PS-DVB-TMG AVEC LE DIGLYME COMME SOLVANT

Essai n° 5

Cet essai a été effectué dans des conditions identiques à celles de l'essai n° 3, à la seule différence que le diglyme remplace le DMF comme solvant :

| | |
|---|---|
| Résine PS-DVB-TMG | 19 g |
| Diglyme | 150 g |
| Acide acrylique | 100 g (1,39 mole) |
| $H_2S$ | 142 g (4,2 moles) |
| Ratio molaire | $\frac{H_2S}{AA} = \frac{3}{1}$ |
| Température de réaction | 60 °C |

A la température de 60 °C, la pression dans l'appareillage est de 20 bars (relatifs).

Essai n° 6

Cet essai a été effectué dans des conditions identiques à celles de l'essai n°4, à la seule différence que le diglyme remplace le DMF comme solvant :

| | |
|---|---|
| Résine PS-DVB-TMG | 19 g |
| Diglyme | 150 g |
| Acide acrylique | 100 g (1,39 mole) |
| $H_2S$ | 284 g (8,34 moles) |
| Ratio molaire | $\frac{H_2S}{AA} = \frac{6}{1}$ |
| Température de réaction | 60 °C |

A la température de 60 °C, la pression dans l'appareillage est de 24 bars (relatifs).

Les résultats des essais n° 3 et n° 4 (résine à fonction guanidine TMG) comparés aux résultats des essais n° 1 et n° 2 (résine IRA 94 à fonction amine tertiaire) montrent que la résine PS-DVB-TMG est nettement plus sélective que la résine IRA 94 pour l'obtention de l'acide mercapto-3 propionique.

A bout de 6 heures, avec une charge de résine PS-DVB-TMG (19 g) plus faible qu'avec la résine IRA 94, on obtient des conversions de l'acide acrylique (AA) de niveau comparable.

En fonction du ratio $\frac{H_2S}{AA}$, on retrouve les mêmes effets sur la sélectivité en acide mercapto-3 propionique, c'est à dire d'un ratio $\frac{3}{1}$ à un ratio à un ratio $\frac{6}{1}$ le gain en sélectivité en MPA est comparable.

Les résultats avec la résine PS-DVB-TMG en utilisant le diglyme comme solvant (essais n° 5 et n° 6) sont nettement moins bons qu'avec le diméthylformamide (essais n° 3 et n° 4). Dans le diglyme la conversion de l'acide acrylique est beaucoup plus lente et la sélectivité en acide mercapto-3 propionique est significativement plus faible que dans le DMF.

Le DMF est un solvant remarquable qui a pour effet d'accroître fortement l'activité et la sélectivité de la résine PS-DVB-TMG, pour la réaction (1) ci-dessus.

Les valeurs de conversion et de sélectivité des essais 1 à 6 précédents ainsi que des essais suivants sont quantitatifs, car étalonnés par des mélanges de référence.

Si on ne tient pas compte des facteurs de réponse chromatographiques, on obtient pour l'essai n°1 une sélectivité apparente en MPA de 80,4 %( au lieu des 68,4 % réels) et en TPDA de 19,3 %( au lieu des 31,2 % réels).

De même, l'essai n°2 conduit à une sélectivité apparente en MPA de 90,9 % (au lieu de 84,1 %) et une sélectivité apparente en TDPA de 9,9 % (au lieu de 15,8 %).

Les résultats obtenus pour les essais ci-dessus sont présentés dans le tableau I suivant.

TABLEAU I

| Essai n° | Solvant | $\frac{H_2S}{AA}$ | 2 heures | | | 4 heures | | | 6 heures | | |
|---|---|---|---|---|---|---|---|---|---|---|---|
| | | | Conv. AA (%) | Sél. MPA (%) | Sél. TDPA (%) | Conv. AA (%) | Sél. MPA (%) | Sél. TDPA (%) | Conv. AA (%) | Sél. MPA (%) | Sél. TDPA (%) |
| 1* | DMF | $\frac{3}{1}$ | 89,8 | 70,9 | 28,8 | 95,7 | 70,4 | 29,3 | 98,6 | 68,4 | 31,2 |
| 2* | DMF | $\frac{6}{1}$ | 95,5 | 84,5 | 15,3 | 97,6 | 84,2 | 15,6 | 99,0 | 84,1 | 15,8 |
| 3 | DMF | $\frac{3}{1}$ | 79,1 | 89,7 | 10,1 | 89,0 | 86,6 | 13,3 | 97,7 | 86,5 | 13,4 |
| 4 | DMF | $\frac{6}{1}$ | 85,5 | 94,7 | 5,2 | 94,6 | 92,9 | 7,0 | 98,2 | 92,5 | 7,4 |
| 5 | Diglyme | $\frac{3}{1}$ | 41,3 | 92,6 | 7,2 | 58,3 | 78,9 | 20,9 | 76,9 | 77,3 | 22,5 |
| 6 | Diglyme | $\frac{6}{1}$ | 61,6 | 88,3 | 11,5 | 78,3 | 86,9 | 12,9 | 89,3 | 85,8 | 14,0 |

* Essai comparatif

II.5.5 ESSAIS COMPARATIFS DANS LE DMF COMME SOLVANT, A LA TEMPERATURE DE 40 °C, DE RESINES A FONCTIONS GUANIDINES (INVENTION) ET DE RESINES A FONCTION AMINE TERTIAIRE.

Ces essais ont été effectués à plus basse température dans le but de comparer les activités catalytiques des résines à fonction guanidine et des résines à fonction amine, dans des conditions cinétiques plus favorables pour l'obtention de l'acide mercapto-3 propionique.

Deux séries d'essais ont été effectuées dans des conditions opératoires identiques, avec des ratios molaires $H_2S$/acide acrylique de $\frac{3}{1}$ et de $\frac{6}{1}$ à partir de 100 g (1,39 mole) d'acide acrylique et de 150 g de DMF (solvant).

Deux résines à fonction amine tertiaire ont été testées:

* IRA 94 (24 g), résine Rohm and Haas donnée comme exemple dans J07,228,568), et utilisée comme résine de référence dans nos essais précédents.
* A-21 (22,5 g) résine Rohm and Haas donnée comme exemple dans US 5.008.432 (ou EP 208323).

Deux résines à fonction guanidine de l'invention ont été testées :

* PS-DVB-TBD (21,6 g), résine à fonction 1,5,7-triaza bicyclo [4.4.0] déc-5-éne préparée selon le mode opératoire décrit ci-dessus.
* PS-DVB-TMG (19 g), résine à fonction 1,1,3,3-tétraméthylguanidine préparée selon le mode opératoire décrit ci-dessus

**Essais n°7,8,9 et 10 à rapport molaire** $\frac{H_2S}{AA} = \frac{3}{1}$

| Conditions : | |
|---|---|
| DMF | 150 g |
| Acide acrylique | 100 g (1,39 mole) |
| $H_2S$ | 142 g (4,2 moles) |

A la température de réaction de 40 °C, la pression dans l'appareillage est de 17 bars (relatifs).

**Essais n° 11,12,13 et 14 à rapport molaire** $\frac{H_2S}{AA} = \frac{6}{1}$

| Conditions : | |
|---|---|
| DMF | 150 g |
| Acide acrylique | 100 g (1,39 mole) |
| $H_2S$ | 284 g (8,34 moles) |

A la température de réaction de 40 °C, la pression dans l'appareillage est de 20 bars (relatifs).

Les résultats obtenus à 40 °C confirment les résultats des essais précédents à 60 °C avec le solvant DMF, à savoir:

* Les résines à fonction guanidine sont plus sélectives en acide mercapto-3 propionique que les résines à fonction amine tertiaire.

Dans le cas des résines à fonction guanidine :

* La diminution de température de 60 °C à 40 °C améliore légèrement la sélectivité en acide mercapto-3 propionique. Par contre la diminution de température affecte la vitesse de conversion de l'acide acrylique.

Dans le cas des résines à fonction amine tertiaire :

* La diminution de température de 60 °C à 40 °C a les mêmes effets que pour les résines guanidine (léger gain de sélectivité en MPA, et baisse de la conversion de l'acide acrylique.
* La résine A-21, plus active que la résine IRA 94, a la même sélectivité en acide mercapto-3 propionique que la résine IRA 94.

Les résultats obtenus pour les 8 résines testées dans ces conditions sont consignés dans le Tableau II.

## TABLEAU II

| Essai n° | Résine | 2 heures | | | 4 heures | | | 6 heures | | |
|---|---|---|---|---|---|---|---|---|---|---|
| | | Conv. AA (%) | Sél. MPA (%) | Sél. TDPA (%) | Conv. AA (%) | Sél. MPA (%) | Sél. TDPA (%) | Conv. AA (%) | Sél. MPA (%) | Sél. TDPA (%) |
| 7* | IRA 94 | 66,9 | 78,2 | 21,6 | 82,5 | 77,8 | 22,0 | 92,5 | 76,9 | 22,9 |
| 8* | A-21 | 81,5 | 78,6 | 21,2 | 93,7 | 77,9 | 21,9 | 97,6 | 75,9 | 23,9 |
| 9 | PS-DVB-TBD | 80,6 | 88,7 | 11,2 | 90,9 | 87,2 | 12,7 | 96,2 | 86,8 | 13,1 |
| 10 | PS-DVB-TMG | 61,1 | 93,6 | 6,3 | 78,5 | 91,3 | 8,6 | 88,3 | 89,2 | 10,7 |
| 11* | IRA 94 | 68,1 | 87,0 | 12,8 | 85,6 | 85,0 | 14,8 | 95,1 | 84,2 | 15,6 |
| 12* | A-21 | 84,1 | 85,6 | 14,2 | 90,5 | 84,5 | 15,3 | 97,4 | 84,1 | 15,7 |
| 13 | PS-DVB-TBD | 86,0 | 94,5 | 5,4 | 93,8 | 93,8 | 6,1 | 96,8 | 92,8 | 7,1 |
| 14 | PS-DVB-TMG | 65,1 | 96,4 | 3,5 | 86,2 | 96,2 | 3,7 | 94,1 | 93,7 | 6,2 |

* Essai comparatif

**Revendications**

1. Procédé de synthèse de l'acide mercapto-3 propionique par réaction d'addition de $H_2S$ à l'acide acrylique en présence d'un support solide possédant des groupes fonctionnels basiques, caractérisé en ce que les groupes fonctionnels sont des groupes guanidine, à la condition que ceux-ci soient dépourvus d'hydrogène directement lié à un atome d'azote.

2. Procédé suivant la revendication 1, caractérisé en ce que ces groupes guanidine sont choisis parmi :

   1°) un radical guanidine de formule générale (C) :

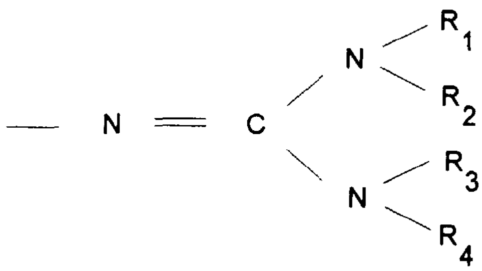

( C)

   dans laquelle $R_1$, $R_2$, $R_3$, $R_4$ sont indépendamment l'un de l'autre des groupes hydrocarbonés tels que méthyle, éthyle, propyle, butyle, l'azote imine étant lié au support solide par une liaison chimique ou une succession de liaisons chimiques,
   2°) un radical guanidine bicyclique de formule (D) :

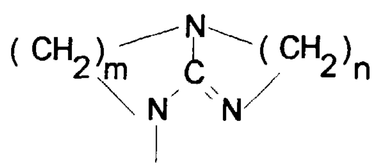

( D)

   dans laquelle m et n valent chacun de 2 à 4, avec la condition que n soit inférieur ou égal à m, ce radical (D) étant lié au support solide par une liaison chimique ou une succession de liaisons chimiques partant de l'azote initialement N-H de la guanidine bicyclique correspondante.

3. Procédé suivant la revendication 2, caractérisé en ce que le radical (D) est choisi parmi les radicaux dérivés des guanidines suivantes: 1,5,7-triazabicyclo [4.3.0] non-6-ène (m = 3, n = 2), 1,5,7-triazabicyclo [4.4.0] déc-5-ène (m = 3, n = 3), 1,6,8-triazabicyclo [5.3.0] déc-7-ène (m = 4, n = 2), 1,4,6-triazabicyclo [3.3.0] oct-4-ène (m = 2, n = 2).

4. Procédé selon la revendication 2 ou 3, caractérisé en ce que le support solide est une résine à base de polystyrène divinylbenzène (PS-DVB) ayant la formule générale (I):

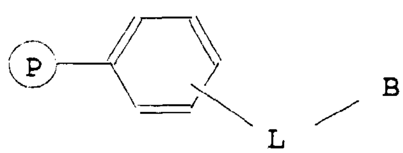

( I)

   B étant un groupe choisi parmi les radicaux de formule générale (C) ou (D), L étant un radical organique linéaire ayant une longueur égale ou supérieure à celle du radical méthylène -(CH2-) et notamment le radical méthylène,

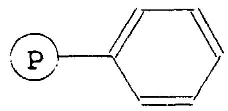

étant le support résine PS-DVB.

5. Procédé suivant la revendication 4, caractérisé en ce que :

- le radical (C) est substitué par L, ce dernier représentant alors un radical -CH2-,et $R_1$, R2, R3 et R4 représentant chacun un groupe méthyle,
- le radical (D) est substitué par L sur l'azote qui, dans le composé bicyclique apparenté, porte un hydrogène, avec la condition que L représente alors un radical-$(CH2)_p$-, p entier valant 1 à 9.

6. Procédé suivant la revendication 4, caractérisé en ce que la résine polymérique a la formule générale (II) :

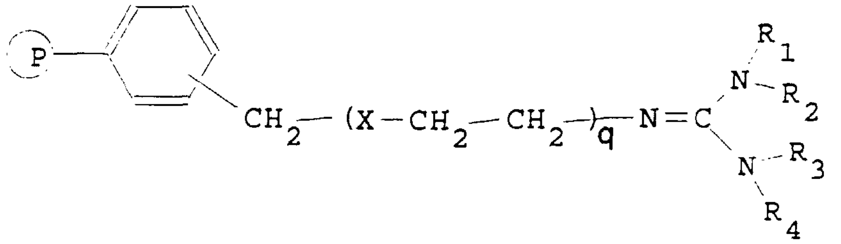
(II)

dans laquelle X représente l'atome d'oxygène ou de soufre, q vaut 1 ou 2, $R_1$, $R_2$, $R_3$ et $R_4$ sont indépendamment l'un de l'autre choisis parmi les groupes méthyle, éthyle, propyle, butyle.

7. Procédé suivant la revendication 6, caractérisé en ce que $R_1$, $R_2$, $R_3$ et R4 représentent chacun un groupe méthyle et q vaut 1.

8. Procédé suivant l'une des revendications 1 à 7, caractérisé en ce que la réaction d'addition a lieu en présence d'un solvant, ce demier n'ayant pas d'hydrogène mobile.

9. Procédé suivant la revendication 8, caractérisé en ce que ledit solvant est un solvant amide, ester, éther ou cétone ou un de leurs mélanges.

10. Procédé suivant la revendication 9, caractérisé en ce que ledit solvant est choisi parmi le diméthylformamide (DMF), le diéthylèneglycol diméthyl éther, le dioxane.

11. Procédé suivant la revendication 10, caractérisé en ce que ledit solvant est le DMF.

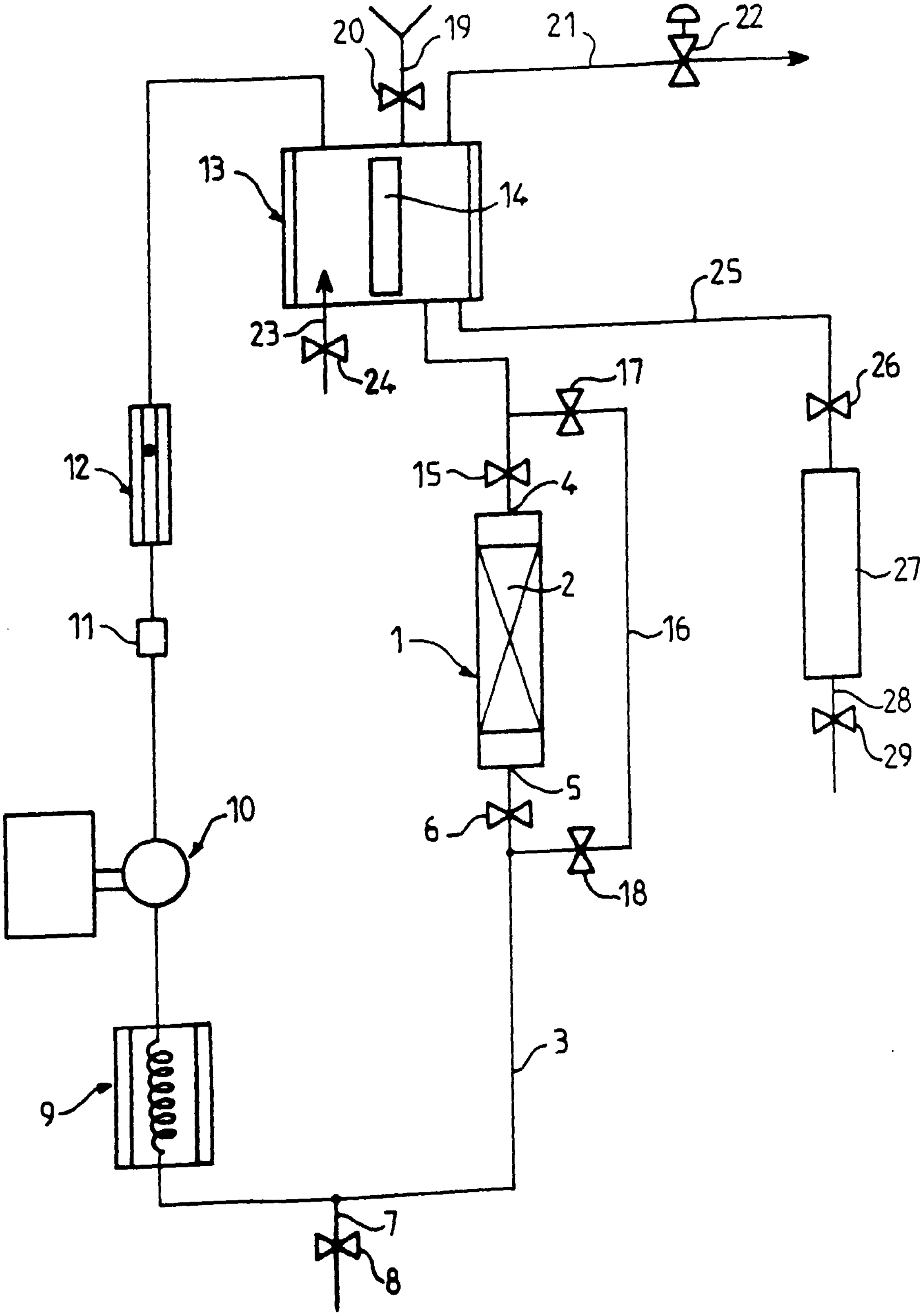
20
19
21
22
13
14
25
23
24
17
26
15
4
12
2
27
11
1
16
28
29
5
10
6
18
3
9
7
8

Office européen des brevets

# RAPPORT DE RECHERCHE EUROPEENNE

Numéro de la demande
EP 97 40 2126

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| A<br><br><br><br><br><br>D | CHEMICAL ABSTRACTS, vol. 124, no. 9, 26 février 1996<br>Columbus, Ohio, US;<br>abstract no. 116667,<br>XP002033492<br>* abrégé *<br>& JP 07 228 568 A (NIPPON CATALYTIC CHEM IND)<br>--- | 1,8-11 | C07C319/04<br>C07C323/52 |
| A,D | US 5 008 432 A (J.S. ROBERTS)<br>* colonne 2, ligne 10 - colonne 3, ligne 60; exemple VII *<br>--- | 1,8 | |
| A,D | K. IIJIMA ET AL:<br>J.M.S. - PURE APPL. CHEM.,<br>vol. A29, no. 3, 1992,<br>pages 249-261, XP000196468<br>* page 249 - page 252 *<br>--- | 1-5 | |
| A | US 4 231 956 A (D.S. SULLIVAN ET AL)<br>* revendications 1,2 *<br>--- | 1,3 | DOMAINES TECHNIQUES RECHERCHES (Int.Cl.6) |
| A,D | US 5 028 259 A (L.-T. W. LIN ET AL)<br>----- | | C07C |

Le présent rapport a été établi pour toutes les revendications

| Lieu de la recherche | Date d'achèvement de la recherche | Examinateur |
|---|---|---|
| BERLIN | 27 octobre 1997 | Van Amsterdam, L |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)